Europäisches Patentamt

European Patent Office

Office européen des brevets

(11) Publication number: **0 140 848**
A1

# (12) EUROPEAN PATENT APPLICATION

(21) Application number: **84850188.8**

(22) Date of filing: **15.06.84**

(51) Int. Cl.⁴: **A 61 K 31/655,** A 61 K 31/60, C 07 C 107/06, C 07 C 107/04

(30) Priority: **15.06.83 SE 8303400**

(43) Date of publication of application: **08.05.85**
**Bulletin 85/19**

(84) Designated Contracting States: **AT BE CH DE FR GB IT LI LU NL SE**

(71) Applicant: **PHARMACIA AB, S-751 82 Uppsala (SE)**

(72) Inventor: **Agback, Karl Hubert, Mimervägen 7, S-754 40 Uppsala (SE)**
Inventor: **Truelove, Sidney Charles, 16 South Street, Oxford OX2 0BE (GB)**

(74) Representative: **Engholm, Carl et al, Pharmacia AB Patent Department Rapsgatan 7, S-751 82 Uppsala (SE)**

(54) **A pharmaceutical composition containing azo-bis-salicylic acid or a salt thereof, and a method for the treatment of inflammatory conditions in the intestines.**

(57) The present invention is concerned with a novel composition for the treatment of inflammatory disorders in the intestine (IBD), especially ulcerative colitis. The composition contains 4,4'-azo-bis-salicylic acid and/or a pharmacologically acceptable and therapeutically active salt thereof.

The invention also provides a novel method for the treatment of inflammatory disorders in the intestine, especially ulcerative colitis.

EP 0 140 848 A1

Azo-bis-salicylic acid and salt thereof, and pharmaceutical
formulations and uses of said acid and salt

This invention is concerned with a novel composition for the
treatment of inflammatory disorders in the intestine,
especially ulcerative colitis.

The composition contains 4,4'-azo-bis-salicylic acid or its
pharmacologically acceptable salts, in particular the
disodium salt. The present invention also provides a novel
method for treating inflammatory disorders in the intestine
(IBD), especially ulcerative colitis. The method comprises
oral or rectal administration of a compound suitably formulated for its contemplated use; e.g. for oral administration
in the form of a tablet or capsule, and for rectal administration in the form of an enema or suppository.

Medical treatments of ulcerative colitis have been carried
out in various different ways. In particular two methods are
now in use to a considerable extent:

Corticosteroids are relied on in severe acute cases, but
they have highly objectionable side effects and are therefore
unsuitable for more general use. Sulfasalazine is the
compound that is being used most generally and most extensively.
It is used both in acute phase treatments and prophylactically
for preventing an acute condition (relapse prevention
effect). Sulfasalazine is believed to exert its effect by
being cleaved to sulfapyridine and 5-aminosalicylic acid
(5-ASA) in the patient's large intestine. The cleavage
product 5-ASA is believed to be responsible for the therapeutical effect, by direct local action on the intestinal
mucosa, while the other cleavage product, sulfapyridine, can
hardly be considered to have any positive effect against the
disease at all but is responsible for most of the side
effects of sulfasalazine treatments. As regards 5-ASA, its
only known side effect is a toxic effect on the kidneys.

0140848

Other methods of treating inflammatory disorders of the intestine comprise rectal or oral treatment with 5-ASA (WP-A-8102671), 5,5'-azo-bis-salicylic acid (A) (EP-A-0036637), carboxyphenylazosalicylic acid (B) (EP-A-0045006) and its carboxymethylamide (C) (GB-A-2080796). It is believed that when the three last-mentioned compounds are administered orally they can be transported to the large intestine where they are then subject to being split in a manner analogous to the splitting of sulfasalazine, to thus form 5-aminosalicylic acid plus another compound: p-aminobenzoic acid or p-aminohippuric acid, respectively. Compounds A, B and C may be considered to act as carriers of 5-ASA during transport through the digestive tract. Splitting of the compound 5,5'-azo-bis-salicylic acid gives only 5-ASA.

Moreover it has been found that 4-aminosalicylic acid, PAS, a well-known anti-tuberculosis agent, may be useful in the treatment of ulcerative colitis by rectal administration (EP-A-62000). Oral administration of PAS or of 5-ASA is not recommendable because both of these substances are absorbed long before reaching that diseased region of the large intestine which is their desired target region for exerting their local effect.

It is known that such splitting of azo compounds in the intestine as will occur with e.g. sulfasalazine will proceed satisfactorily as long as the azo compound contains a hydroxy group in position para or ortho to an azo group. Upon chemical reduction of azo compounds containing such hydroxy groups, the split amines are formed immediately. If the azo compound does not contain hydroxy groups in ortho or para position (note that amino groups give the same effect), reduction will proceed with the formation of stable hydrazo compounds. Much more vigorous reductive conditions are then required for further reducing the hydrazo compounds to form amines.

The compund 4,4'-azo-bis-salicylic acid was once considered to be potentially effective against tuberculosis. However, this compound was shown to be inactive against that disease, according to pharmacological testing (Ind. J. Med. Res. 40 (1952), p. 4-5, and Ind. J. Pharm. 13 (1951), p. 3-5).

These early results moreover show that under the conditions employed in the in vivo tests no (or very little) free PAS can have formed by splitting: If it had formed, the results obtained ought to have been good ones, that is, the PAS if it had formed would have shown a very good tuberculostatic effect in the animal model chosen. There is nothing remarkable about the results actually obtained, inasmuch as splitting of 4,4'-azo-bis-salicylic acid to PAS was not to be expected because of the greater resistance of the 4,4' compound to reduction as compared to for example the isomeric 5,5'-azo-bis-salicylic acid.

Surprisingly, it has now been found that in contrast to the evidence provided by earlier results the 4,4'-azo-bis-salicylic acid (as well as salts thereof) is split reductively in vivo, with the formation of PAS. The reduction takes place in the large intestine or corresponding organ under the action of the reducing conditions of the local environment. The splitting is a necessary prerequisite for the 4,4'-azo-bis-salicylic acid to become effective as an agent against the intestinal disorders, and the present invention is based on the highly surprising discovery that splitting actually does take place. Corroboration of this discovery is illustrated in Example 5.

The invention relates to compositions which are novel for treating inflammatory disorders in the intestine. In addition to a therapeutically active amount of 4,4'-azo-bis-salicylic acid and/or a salt thereof which is pharmaceutically acceptable and therapeutically active, the compositions according to the invention may optionally contain a pharmaceutically acceptable carrier and/or a suitable adjuvant. Examples of suitable salts include salts of said acid with pharmaceutically acceptable alkali or alkaline earth metals, and salts with positively charged and pharmaceutically acceptable carriers.

The compositions according to the invention may take the form of tablets, dragées, capsules, enemas etc. They are produced in a known per se manner in that 4,4'-azo-bis-salicylic acid is mixed with the desired carrier material and/or appropriate auxiliary substances to thus give a suitable galenic form. For example, the composition may be coated with a film that is resistant to gastric juice and is dissolved only after it has reached the intestine. When a composition according to the invention is prepared the free acid may be replaced by one or more of its salts which are suitable for the purpose in question. The amount of acid or its salt per dosage unit will vary, depending in each case on inter alia the number of administrations per day, and the individual and total dosages per day.

By analogy with what is known about 5-aminosalicylic acid, it is expected that only a minor portion of the PAS and Ac-PAS as formed will be absorbed from the large intestine and secreted in the urine.

The release of PAS moreover proceeds relatively slowly, which is an advantage especially in cases where the site of the inflammatory intestinal disorder is in the lower portion of the large intestine. The toxicity of 4,4'-azo-bis-salicylic acid, PAS and Ac-PAS is remarkably low.

Therefore, 4,4'-azo-bis-salicylic acid and its pharmacologically tolerable salts are potentially useful agents for the treatment of inflammatory disorders of the intestine, in particular ulcerative colitis.

The present invention is also concerned with a novel method of treating inflammatory conditions in the intestine, especially in the large intestine, and in particular ulcerative colitis. The method is charactexized by rectally or orally administering a therapeutically active dose of 4,4'-azo-bis-salicylic acid or a salt thereof which is pharmaceutically acceptable and therapeutically active. This of course also includes the possibility of administering the free acid together with one or more of such salts.

According to a preferred embodiment, the acid is administered in salt form, and such a salt may advantageously be an alkali or alkaline earth metal salt, e.g. sodium salt of the acid. Administration is usually performed together with a pharmaceutically acceptable carrier and/or a suitable adjuvant.

The daily dose of the active substance, 4,4'-azo-bis-salicylic acid, will vary from case to case and may amount to for instance about 0,1 to about 10 g per day and adult weighing 75 kg. In normal therapy cases, a preferred dosage will as a rule give a good therapeutical effect while at the same time side effects are kept at an acceptable low level. The total daily dose may be portioned out among a plurality of administrations per day, for instance 1 to 5 administrations. Where the drug is administered in the form of the salt of the acid, the doses mentioned above refer to the corresponding amount of free 4,4'-azo-bis-salicylic acid.

Production of 4,4'-azo-bis-salicylic acid is known per se. The compound may thus be produced according to e.g. Budziarek, R. et al. (J. Chem. Soc. (1955), part III p. 3158-63). In accordance with this method methyl 4-aminosalicylate is subjected to an oxidative self-coupling reaction so as to form 3,3'-dihydroxy-4,4'-dimethoxycarbonyl-azooxybenzene. The azooxy group of the coupling reaction product is reduced whereupon the methyl ester groups are hydrolyzed to thus form the desired 4,4'-azo-bis-salicylic acid. It will be appreciated of course that also other types of syntheses may be chosen, but an important point to be noted is that the synthesis method chosen should give a product of such purity as to be pharmaceutically acceptable. This "pharmaceutically acceptable degree of purity" means that the product must not contain impurities which give rise to unacceptable side effects. Normally an absolute purity of 98 % (w/w) is suitable in this respect, the product having at the same time to fulfill the ordinary quality requirements of medicines.

A number of non-limitative working examples are set out below.

## Example 1

| | |
|---|---|
| 4,4-azo-bis-salicylic acid (produced according to J. Chem. Soc (1955) part III p. 3158-63) | 250 mg |
| starch | 125 mg |
| Mg stearate | 20 mg |
| Aerosil½ 200 (silica particles, Degussa, Federal Republic of Germany) | 5 mg |

are mixed homogeneously. Water is added, whereupon the mixture is granulated at 60 $^{o}$C and compressed to form tablets weighing 400 mg.

If desired the tablet may be provided in a manner known per se with a coating of a film that is soluble in the stomach or in the small intestine.

## Example 2

| | |
|---|---|
| disodium 4,4'-azo-bis-salicylate | 250 mg |
| starch | 110 mg |
| Mg stearate | 15 mg |

are mixed homogeneously and compressed to form tablets weighing 375 mg. Disodium 4,4'-azo-bis-salicylic acid is produced in a manner known per se by means of acidifying an alkaline aqueous solution of 4,4'-azo-bis-salicylic acid to pH 6-7.

Example 3

The pH of a 10.0 lit. aqueous solution containing 100 g of disodium 4,4'-azo-bis-salicylate is adjusted to pH 7,5, whereupon the solution is filled into 100 ml bottles for rectal administration. The bottles are then sterilized.

Example 4

Gelatin capsules containing 200 mg of disodium 4,4'-azo-bis-salicylate are produced by automatic filling with crystalline salt of particle size 0.15 - 2.0 mm obtained by sieve screening.

Example 5

Splitting of 4,4'-azo-bis-salicylic acid in the intestine

After a single dose of 100 mg/kg given orally to mice, urine was collected during a period of 24 hours. The amount of p-aminosalicylic acid (PAS and Ac-PAS, that is, N-acetyl-p-aminosalicylic acid) present in the urine was measured by means of chemical analysis (Willonghby, C.P. et al.; but 23 (1982) p. 1081 - 87). The results are set forth in the table below.

0140848

Amount secreted, % of dose administered

|  | Ac-PAS | PAS |
|---|---|---|
| Mouse No. 1 | 10 | 0.3 |
| 2 | 15 | 3.2 |
| 3 | 8 | 0.3 |
| 4 | 12 | 0.8 |
| Average | 11.3 | 1.2 |

0140848

## C L A I M S

1. A Composition for oral or rectal treatment of inflammatory conditions in the intestine, comprising a therapeutically active amount of 4,4'-azo-bis-salicylic acid or a therapeutically active and pharmaceutically acceptable salt thereof, and optionally a pharmaceutically acceptable carrier and/or a pharmaceutically adjuvant.

2. A method of treating inflammatory conditions in the intestine, comprising administering to a patient suffering from such condition a therapeutically active amount of 4,4'-azo-bis-salicylic acid or a therapeutically active amount of a pharmaceutically and therapeutically acceptable salt thereof.

-.-.-.-.-.-.-.-.-.-.-

0140848
Application number

European Patent Office

**PARTIAL EUROPEAN SEARCH REPORT**
which under Rule 45 of the European Patent Convention
shall be considered, for the purposes of subsequent
proceedings, as the European search report

EP 84 85 0188 .8

## DOCUMENTS CONSIDERED TO BE RELEVANT

| Category | Citation of document with indication, where appropriate, of relevant passages | Relevant to claim | CLASSIFICATION OF THE APPLICATION (Int. Cl. ³) |
|---|---|---|---|
| D,A | EP-A-0 045 006 (HENNING BERLIN GmbH CHEMIE-UND PHARMAWERK) *Claims 1-4; pages 1-2; examples 2-4* | 1 | A 61 K 31/655<br>A 61 K 31/60<br>C 07 C 107/06<br>C 07 C 107/04 |
| X | DE-A-3 149 359 (G.D. SEARLE & CO) *Claims 1-4; page 9, lines 1-20* | 1 | |
| D,X | EP-A-0 036 637 (PHARMACIA AB) *The whole document* | 1 | |
| D,X | INDIAN JOURNAL OF MEDICAL RESEARCH, vol. 40, January 1952, pages 1-5, Kasauli (IN); J.C. SURI:"Anti-tubercular activity of organic (synthetic) compounds related to PAS in vitro". *Pages 2 and 4* | 1 | |
| | --- -/- | | |

TECHNICAL FIELDS SEARCHED (Int. Cl. ³)

A 61 K
C 07 C

### INCOMPLETE SEARCH

The Search Division considers that the present European patent application does not comply with the provisions of the European Patent Convention to such an extent that it is not possible to carry out a meaningful search into the state of the art on the basis of some of the claims.

Claims searched completely: 1
Claims searched incompletely:
Claims not searched: 2
Reason for the limitation of the search: Method for treatment of the human or animal body by surgery or therapy (see art 52 (4) of the European Patent Convention).

| Place of search | Date of completion of the search | Examiner |
|---|---|---|
| STOCKHOLM | 07-09-1984 | HJÄLMDAHL M. |

European Patent
Office

**PARTIAL EUROPEAN SEARCH REPORT**

| | DOCUMENTS CONSIDERED TO BE RELEVANT | | CLASSIFICATION OF THE APPLICATION (Int. Cl.³) |
|---|---|---|---|
| Category | Citation of document with indication, where appropriate, of relevant passages | Relevant to claim | |
| D,A | JOURNAL OF THE CHEMICAL SOCIETY, 1955, pages 3158-3163, London (GB); R. BUDZIAREK et al: "4-alkyl-3:5--dioxo-1:2-diphenylpyrazolidine derivatives". *Page 3161, the ninth paragraph* | 1 | |
| | --- | | |
| X | GUT, vol. 23, no. 12, December 1982, pages 1081-1087, London (GB); C.P. WILLOUGHBY et al:"Distribution and metabolism in healthy volunteers of disodium azodisalicylate, a potential therapeutic agent for ulcerative colitis". *Pages 1081-1082* | 1 | TECHNICAL FIELDS SEARCHED (Int. Cl.³) |
| | ---- | | |

EPO Form 1505.3   06.78